# EUROPÄISCHE PATENTSCHRIFT

(11) **EP 3 041 443 B1**
(45) Veröffentlichungstag und Bekanntmachung des Hinweises auf die Patenterteilung: **09.05.2018**
(21) Anmeldenummer: 15718130.6
(22) Anmeldetag: 11.03.2015
(51) Int. Cl.: A61F 9/007

(54) **CHIRURGISCHES INSTRUMENT**
SURGICAL INSTRUMENT
INSTRUMENT CHIRURGICAL

(30) Priorität: 11.03.2014 DE 102014204480; 14.11.2014 DE 102014223304
(43) Veröffentlichungstag der Anmeldung: 13.07.2016
(73) Patentinhaber: Geuder AG, 69126 Heidelberg (DE)
(72) Erfinder: DRAHEIM, René, 69207 Sandhausen (DE); FRAUENFELD, Dieter, 69121 Heidelberg (DE); GEUDER, Volker, 69126 Heidelberg (DE)
(74) Vertreter: Patent- und Rechtsanwälte Ullrich & Naumann
(86) Internationale Anmeldenummer: PCT/DE2015/200144
(87) Internationale Veröffentlichungsnummer: WO 2015/135544

(56) Entgegenhaltungen:
- EP-A1- 0 919 210
- US-A- 5 257 988
- US-A1- 2005 135 776
- US-A1- 2010 106 054
- US-A1- 2012 035 425

## Beschreibung

Die Erfindung betrifft ein chirurgisches Instrument, insbesondere Vitrektor, mit einem Außenrohr und einem Innenrohr, wobei die beiden Rohre funktional zusammenwirken.

Grundsätzlich geht es hier um ein chirurgisches Instrument, insbesondere um ein Schneidinstrument zur Entnahme von Gewebe. Mit dem Instrument lässt sich das Gewebe - am bzw. im Körper - schneiden und vom Körper bzw. aus dem Körper heraus absaugen. Im Konkreten kann es sich dabei um ein ganz besonderes Schneidinstrument handeln, mit dem im Rahmen der Vitrektomie der Glaskörper im Auge zerstört bzw. zerkleinert und aus dem Auge entfernt wird. Auch lassen sich mit diesem Instrument Blut, Blutgerinnsel und bindegewebsähnliche Veränderungen sowie Bereiche der Netzhaut im Rahmen eines Netzhautpeelings entfernen.

Zum Stand der Technik sei lediglich beispielhaft auf die US 5,630,827 verwiesen, aus der ein für Glaskörperoperationen geeignetes Schneidinstrument bekannt ist. Am proximalen Ende ist das Schneidgerät offen. Am distalen Ende besitzt es eine geschlossene Außenhülse, die in der Nähe des geschlossenen Endes mit mehreren Öffnungen versehen ist. Die Öffnungen bilden jeweils einen Schlitz und ermöglichen das Eintreten von Glaskörpergewebe in das Lumen der Außenhülse. Außerdem besitzt das Schneidinstrument eine konzentrisch zur äußeren Hülse angeordnete innere Hülse, die in der äußeren Hülse längsbeweglich aufgenommen ist und an ihrem distalen Ende eine scharfe Kante zum Schneiden des Glaskörpergewebes beim Vorschieben der Innenhülse umfasst.

Bei der aus der US 5,630,827 bekannten Vorrichtung ist wesentlich, dass die innere Röhre an ihrem freien vorderen Ende eine offene umlaufende Kante aufweist, die als Schneidkante dient. Entsprechend ist diese Kante geformt bzw. geschliffen. Eine schneidende Wirkung kommt dem äußeren Randbereich zu, so dass hier fortan von einer äußeren Schneidkante der inneren Röhre die Rede ist. Diese äußere Schneidkante wirkt mit einer oder mehreren inneren Schneidkanten der äußeren Röhre zusammen, so dass in die Vorrichtung eingedrungenes Gewebe beim gegenseitigen Vorbeigleiten der Schneidkanten geschnitten bzw. abgetrennt wird. Danach kann das Gewebe aus dem Inneren der Vorrichtung, genauer gesagt durch die innere Röhre hindurch, abgesaugt werden.
Aus der WO 98/52502 ist ebenfalls ein gattungsbildendes Schneidinstrument zur Entnahme von Glaskörpergewebe bekannt. Am distalen Ende des Instruments ist eine geschlossene Außenhülse vorgesehen, die in der Nähe des distalen Endes mehrere Schlitze aufweist, durch die Glaskörpergewebe eindringen kann. Konzentrisch zur Außenhülse ist eine längsbewegliche Innenhülse vorgesehen, die an ihrem distalen Ende eine scharfe Kante zum Schneiden des eingedrungenen Glaskörpergewebes besitzt. Außerdem ist ein Antriebsmechanismus für die Innenhülse vorgesehen, durch den die Innenhülse mit ihrer Schneidkante im Bereich des distalen Endes über die in der Außenhülse angeordneten Schlitze hinweg bewegbar ist, so dass dort jeweils ein Schneidvorgang stattfindet.
Die bekannten Vorrichtungen sind jedoch in der Praxis insoweit problematisch, als es regelmäßig erforderlich ist, durch ein separates Gerät die Eingriffsstelle mit Licht zu versorgen, insbesondere bei einem Eingriff in das menschliche Auge.

Des Weiteren ist aus der US 2012/0035425 A1 ein chirurgisches Instrument bekannt, welches auch als Lichtversorgung zur Beleuchtung der Operationsstelle dient. Im konkreten weist das instrument ein Außenrohr auf, dessen Außenwandung von einer spritzgusstechnisch aufgetragenenen Lichtleithülle umschlossen ist.
Die voranstehende Aufgabe ist durch die Merkmale des Anspruchs 1 gelöst, nämlich dadurch, dass das Innenrohr aus einem Licht leitenden Material besteht oder durch ein solches Material beschichtet ist, und zur Lichtleitung vom proximalen Ende zum oder in den Bereich des distalen Endes dient. Am vorderen Ende des Innenrohrs ist der Lichtaustritt definiert, so dass eine Beleuchtung der Operationsstelle stattfindet.

Erfindungsgemäß ist erkannt worden, dass das chirurgische Instrument in idealer Weise auch zur Lichtversorgung genutzt werden kann, nämlich dann, wenn das Innenrohr aus einem Licht leitenden Material besteht. Ebenso ist es denkbar, dass das Innenrohr mit einem Licht leitenden Material beschichtet ist. Jedenfalls dient das Innenrohr zur Lichtleitung vom proximalen Ende, wo das Licht in das Innenrohr eingekoppelt wird, zum distalen Ende, zumindest aber in den Bereich des distalen Endes. Dort kann dann das Licht zu Beleuchtungszwecken der Operationsstelle austreten.

Bei dem Licht leitenden Material kann es sich um Glas, Polymethylmethacrylat, um Polycarbonat, etc. handeln. Sämtliche lichtleitenden Materialien kommen hier in Frage, jedenfalls dann, wenn sie die erforderliche Härte aufweisen, damit das Innenrohr, zumindest im Bereich einer Ausnehmung, schneidende Eigenschaften hat. Eine entsprechende Härte ist erforderlich.

Wie bereits zuvor erwähnt, kann es sich bei dem hier in Rede stehenden Instrument um einen Vitrektor mit einem Außenschneidrohr und einem darin axial laufenden Innenschneidrohr handeln. Das Außenschneidrohr hat mindestens eine Öffnung, die eine Schneidkante bildet. Zumindest das vordere Ende des Innenschneidrohrs wirkt mit der Schneidkante des Außenschneidrohrs zusammen, so dass eine Art Scherenfunktion realisiert ist.

Erfindungsgemäß ist am vorderen Ende des Innenrohrs der Lichtaustritt definiert, so dass eine Beleuchtung der Operationsstelle - in situ - stattfindet.

Bei dem hier in Rede stehenden Vitrektor kann das distale Ende des Außenschneidrohrs geschlossen sein. Auch dort ist ein definierter Lichtaustritt möglich. In besonders vorteilhafter Weise kann am distalen Ende ein Glaskörper, vorzugsweise in Form einer Linse, zum definierten Lichtaustritt ausgebildet sein.

Auch ist es denkbar, dass der aus Glas oder einem sonstigen Licht leitenden Material bestehende Körper -Innenrohr - durch ein den Lichtaustritt verhindertes Material beschichtet ist, welches gleichermaßen zur Stabilisierung bzw. Festigung des Innenrohrs dient. An definierten Stellen, wo nämlich der Lichtaustritt gewünscht ist, ist entsprechend keine Beschichtung vorgesehen und kann dass Licht daher entsprechend der Beugung und Brechung austreten.

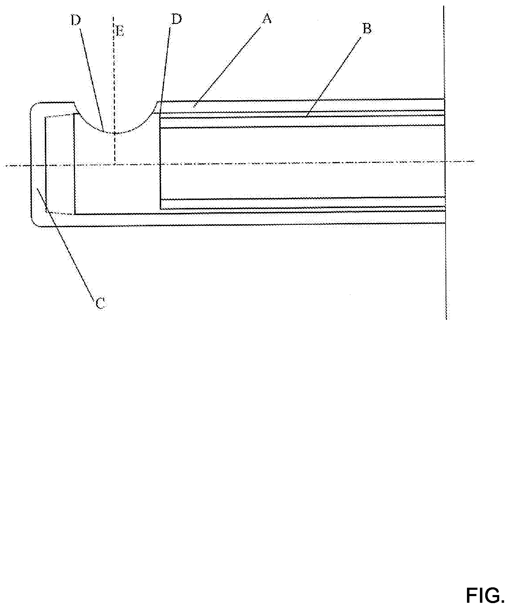

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigt die
- einzige Fig.: in schematischer Ansicht, teilweise, ein Ausführungsbeispiel eines erfindungsgemäßen Instruments am Beispiel eines Vitrektors.

Die einzige Figur zeigt ein chirurgisches Instrument am Beispiel eines Vitrektors. Es ist ein Außenschneidrohr A und ein Innenschneidrohr B vorgesehen, wobei zumindest das Innenschneidrohr B aus Glas oder einem sonstigen Licht leitenden Material bestehen kann. Alternativ ist es denkbar, dass zumindest das Innenschneidrohr mit einer Licht leitenden Beschichtung versehen ist. In diesem Falle handelt es sich bei den Schneidrohren um konventionelle Schneidrohre, die mit Licht leitender Beschichtung ausgestattet sind.

Das Außenschneidrohr A und das Innenschneidrohr B wirken im Bereich einer im Außenschneidrohr A vorgesehenen Öffnung E zusammen, nämlich über deren scharfen Schneidkanten D. Die Schneidkanten D können unter verschiedenen Freiwinkeln definiert sein.

Besteht zumindest das Innenschneidrohr B insgesamt aus einem Licht leitenden Material, ist es von weiterem Vorteil, wenn die Schneidrohre A, B durch ein Licht undurchlässiges Material beschichtet sind, um einen ungewollten Lichtaustritt zu vermeiden. Ein Lichtaustritt kann im Bereich der Schneidöffnung E definiert vorgesehen sein, nämlich im Bereich der Schneidkanten D.

Auch ist es denkbar, dass das Licht leitende Material in spezielle Nutungen eingeführt, gegossen, gespritzt oder sonst wie durch Beschichtung erzeugt ist. Wesentlich ist jedenfalls, dass im distalen Ende des Außenschneidrohs A, beispielsweise im Bereich der Öffnung E, ein definierter Lichtaustritt vorgesehen ist.

Weiter ist es denkbar, dass der Lichtaustritt im Bereich einer endseitigen Linse mit unterschiedlicher Definition des Lichtaustritts - Lichtkegel, Spot, Weitwinkel, etc. am distalen Ende über eine Linse C stattfindet.

Besteht zumindest das Innenschneidrohr B insgesamt aus Licht leitendem Material, sorgt eine Beschichtung bzw. ein Coating dafür, dass der Lichtaustritt nur am distalen Ende C oder im Bereich der Schneidöffnung E stattfindet. Das Coating kann die weitere Aufgabe haben, das Glas zu stabilisieren, beispielsweise es auch in einem gewissen Umfange dehnbar oder biegbar zu machen.

Hinsichtlich weiterer vorteilhafter Ausgestaltungen des erfindungsgemäßen chirurgischen Instruments wird zur Vermeidung von Wiederholungen auf den allgemeinen Teil der Beschreibung sowie auf die beigefügten Patentansprüche verwiesen.

Schließlich sei ausdrücklich darauf hingewiesen, dass das voranstehend beschriebene Ausführungsbeispiel des erfindungsgemäßen chirurgischen Instruments lediglich zur Erörterung der beanspruchten Lehre dient, diese jedoch nicht auf das Ausführungsbeispiel einschränkt.

## Patentansprüche

1. Chirurgisches Instrument, insbesondere Vitrektor, mit einem Außenrohr und einem Innenrohr, wobei die beiden Rohre funktional zusammenwirken,
**dadurch gekennzeichnet, dass** das Innenrohr aus einem Licht leitenden Material besteht oder durch ein solches Material beschichtet ist und zur Lichtleitung vom proximalen Ende zum oder in den Bereich des distalen Endes dient, wobei am vorderen Ende des Innenrohrs der Lichtaustritt definiert ist, für eine Beleuchtung einer Operationsstelle.

2. Instrument nach Anspruch 1, **dadurch gekennzeichnet, dass** es sich bei dem Licht leitenden Material um Glas, PMMA, Polycarbonat, etc. handelt.

3. Instrument nach Anspruch 1 oder 2, wobei es sich bei dem Instrument um einen Vitrektor mit einem Außenschneidrohr und einem darin axial beweglichen Innenschneidrohr handelt und mindestens eine Öffnung eine Schneidkante bildet, **dadurch gekennzeichnet, dass** im Bereich der Schneidkante ein Lichtaustritt definiert ist.

4. Instrument nach Anspruch 3, wobei das Außenschneidrohr am distalen Ende geschlossen ist, **dadurch gekennzeichnet, dass** das distale Ende einen Lichtaustritt definiert.

5. Instrument nach einem der Ansprüche 1 bis 4, **dadurch gekennzeichnet, dass** am distalen Ende ein Glaskörper, vorzugsweise in Form einer Linse, zum definierten Lichtaustritt ausgebildet ist.

6. Instrument nach einem der Ansprüche 1 bis 5, **dadurch gekennzeichnet, dass** das lichtleitende Material des Außenrohrs und/oder des Innenrohrs zur Verhinderung eines ungewollten Lichtaustritts beschichtet und lediglich an definierten Lichtaustrittsstellen unbeschichtet ist.

## Claims

1. Surgical instrument, in particular vitrector, having an outer pipe and an inner pipe, wherein the two pipes cooperate in a functional manner,
**characterised in that** the inner pipe comprises a light-conducting material or is coated with such a material and serves to conduct light from the proximal end to or into the region of the distal end, wherein at the front end of the inner pipe the light emission is defined for illuminating an operation location.

2. Instrument according to claim 1, **characterised in that** the light-conducting material is glass, PMMA, polycarbonate, etcetera.

3. Instrument according to claim 1 or 2, wherein the instrument is a vitrector having an outer cutting pipe and an inner cutting pipe which is axially movable therein and at least one opening forms a cutting edge, **characterised in that** a light emission is defined in the region of the cutting edge.

4. Instrument according to claim 3, wherein the outer cutting pipe is closed at the distal end, **characterised in that** the distal end defines a light emission.

5. Instrument according to any one of claims 1 to 4, **characterised in that**, at the distal end, a glass member, preferably in the form of a lens, is formed for the defined light emission.

6. Instrument according to any one of claims 1 to 5, **characterised in that** the light-conducting material of the outer pipe and/or the inner pipe is coated to prevent undesirable light emission and is uncoated only at defined light emission locations.

## Revendications

1. Instrument chirurgical, plus particulièrement un dispositif de vitrectomie, avec un tube externe et un tube interne, les deux tubes interagissant de manière fonctionnelle,
**caractérisé en ce que** le tube interne est constitué d'un matériau conduisant la lumière ou est revêtu d'un tel matériau et permet la conduction de lumière à partir de l'extrémité proximale ou au niveau de l'extrémité distale, la sortie de lumière étant définie au niveau de l'extrémité avant du tube interne, pour un éclairage d'un champ opératoire.

2. Instrument selon la revendication 1, **caractérisé en ce que** le matériau conduisant la lumière est du verre, du PMMA, du polycarbonate, etc.

3. Instrument selon la revendication 1 ou 2, l'instrument étant un dispositif de vitrectomie avec un tube de coupe externe et d'un tube de coupe interne mobile axialement et au moins une ouverture comporte une arête de coupe, **caractérisé en ce qu'**une sortie de lumière est définie au niveau de l'arête de coupe.

4. Instrument selon la revendication 3, le tube de coupe externe étant fermé au niveau de l'extrémité distale, **caractérisé en ce que** l'extrémité distale définit une sortie de lumière.

5. Instrument selon l'une des revendications 1 à 4, **caractérisé en ce que**, au niveau de l'extrémité distale, est prévu un corps en verre, de préférence sous la forme d'une lentille, pour la sortie de lumière définie.

6. Instrument selon l'une des revendications 1 à 5, **caractérisé en ce que** le matériau conduisant la lumière du tube externe et/ou du tube interne est revêtu pour empêcher une sortie de lumière indésirable et n'est pas revêtu uniquement à des endroits de sortie de lumière définis.
